# EUROPÄISCHE PATENTSCHRIFT

(11) **EP 3 141 203 B1**
(45) Veröffentlichungstag und Bekanntmachung des Hinweises auf die Patenterteilung: **20.04.2022**
(21) Anmeldenummer: 15184682.1
(22) Anmeldetag: 10.09.2015
(51) Int. Cl.: A61B 18/04, A61B 18/14

(54) **ABLATIONSEINRICHTUNG ZUR GROSSFLÄCHIGEN MUKOSAABLATION**
ABLATION DEVICE FOR LARGE-SCALE MUCOSA ABLATION
DISPOSITIF D'ABLATION DE MUQUEUSE DE GRANDE ETENDUE

(43) Veröffentlichungstag der Anmeldung: 15.03.2017
(73) Patentinhaber: Erbe Elektromedizin GmbH, 72072 Tübingen (DE)
(72) Erfinder: Enderle, Markus, 72070 Tübingen (DE); Fischer, Klaus, 72202 Nagold (DE); Stäbler, Thomas, 72070 Tübingen (DE)
(74) Vertreter: Rüger Abel Patentanwälte PartGmbB

(56) Entgegenhaltungen:
- WO-A1-00/09053
- WO-A1-2008/090004
- DE-A1- 10 000 091
- US-A1- 2011 184 408
- None

## Beschreibung

Die Erfindung betrifft eine Ablationsvorrichtung insbesondere für die Mucosaablation.

Die therapeutische Mucosaablation, z.B. zur Tumorablation oder Zerstörung von das Essverhalten beeinflussenden Zellen der Magenwand, vorteilhafthafterweise zur Gewichtsreduktion, wird typischerweise durch endoskopische Intervention durchgeführt, wobei zur Ablation spezielle Sonden zur Anwendung kommen können.

Beispielsweise ist dazu aus der WO 2011/022069 A ein Endoskop mit einer Endkappe bekannt, die auf die Mucosa aufzusetzen ist und in deren Innenraum eine Argonplasmakoagulation durchgeführt wird. Die Kappe soll den Einwirkungsbereich der Argonplasmakoagulation begrenzen und somit die Mucosakoagulation definieren.

Aus der WO 2008/09004 A1 ist ein bipolares Instrument zur elektrochirurgischen Behandlung von Gewebe bekannt. Dieses Instrument weist zumindest in einer der beschriebenen Ausführungsformen einen Sondenkörper mit zwei zueinander parallel geführten Gaskanälen auf, wobei in jedem der beiden Kanäle eine Elektrode angeordnet ist. Die Elektroden sind dazu eingerichtet und vorgesehen, zwischen einander einen Lichtbogen auszubilden. Dieser wird durch den von den beiden Gaskanälen geführten Gasströmen zu dem biologischen Gewebe hin geblasen. Zur Verlängerung des Lichtbogens können die Enden der Elektroden voneinander weggebogen sein, so dass sie divergieren.

Aus der US 8 641 711 B2 ist ein Instrument zur Abtragung von Gewebeschichten von Hohlorganen bekannt, wobei das Instrument einen elektrisch aktiven Kopf mit Elektroden aufweist, an dem ein expandierbares Element vorgesehen ist, um den Kopf des Instruments in Bezug auf die gegenüber liegende Gewebewand definiert zu positionieren. Dieses Instrument setzt Hohlorgane mit beschränktem Durchmesser voraus, wie es zum Beispiel im Darm der Fall ist.

Die großflächige Mucosaablation stellt an den Behandler besondere Herausforderungen hinsichtlich Geduld und Fingerfertigkeit. Dies insbesondere, wenn dazu für den allgemeinen Gebrauch vorgesehene flexible Instrumente, wie Polypektomieschlingen oder dergleichen, eingesetzt werden. Mit solchen Schlingen wird in einem Arbeitsschritt lediglich eine Resektion der Mucosa von ca. 2 cm² ausgeführt. Im Fundus- und Cardia-Bereich des Magens ist eine Resektion 'mit Hilfe eines flexiblen Endoskops sehr schwierig. Außerdem besteht das Risiko der Perforation.

Bei der Ablation der Mucosa ist eine nicht ausreichende Ablation ebenso abzulehnen wie eine zu tiefe Einwirkung, weil dadurch darunter liegende Gewebeschichten geschädigt werden könnten, bis hin zur Perforation des Magens.

Es besteht demnach der Wunsch nach einem Instrument und einem Verfahren, nach dem sich die Mucosaablation prozesssicher und gegebenenfalls über größere Flächen erstreckend und in guter Qualität durchführen lässt.

Diese Aufgabe wird mit dem Instrument nach Anspruch 1 gelöst.

Die erfindungsgemäße Ablationsvorrichtung weist die Merkmale des Anspruchs 1 auf. Die Kanäle und die Elektroden schließen miteinander einen spitzen Winkel ein. Dieser liegt vorzugsweise zwischen 10° und 60°, vorzugsweise beträgt er 10° bis 30°, weiter vorzugsweise 25°. Die Kanäle sind Gasleitungskanäle, durch die geeignetes Gas, wie beispielsweise Argon, geleitet werden kann. Damit kann mit Hilfe von den Elektroden ausgehender Funken ein Argon-Plasma erzeugt werden, das zu einer Koagulation des vor den Elektroden liegenden Gewebes genutzt werden kann. Obwohl die Ablationsvorrichtung wenigstens zwei Elektroden aufweist, ist sie vorzugsweise ein monopolares Instrument. Dies bedeutet, dass die Neutralelektrode nicht an dem Instrument vorgesehen ist, sondern großflächig an dem Patienten befestigt wird. Der Stromfluss findet somit zwischen dem biologischen Gewebe und den Elektroden der Ablationsvorrichtung statt.

Durch die Anordnung der Kanäle und durch die Anordnung der Elektroden in einem spitzen Winkel zueinander kann erreicht werden, dass die von den beiden Kanälen ausgehenden Plasmaströme divergieren und einen breiten streifenförmigen Bereich der Mucosa koagulieren, wenn die Ablationsvorrichtung entlang der Mucosa bewegt wird. Die aus den beiden Kanälen austretenden Plasmaströme können gemeinsam einen etwa fächerförmigen Strom bilden, d.h. einen Strahl mit nichtkreisförmigem (z.B. ovalem oder streifenartigem) Querschnitt. Die Austrittsöffnungen der beiden Kanäle liegen vorzugsweise auf einer Linie, die quer zur Bewegungsrichtung der Ablationsvorrichtung orientiert ist. Bei geeigneter Festlegung des Winkels zwischen den beiden Kanälen und/oder zwischen den beiden Elektroden sowie der Ausströmgeschwindigkeit des Gases aus den Kanälen, der Gasmenge sowie der elektrischen Beaufschlagung der beiden Elektroden kann erreicht werden, dass sich über die Breite des koagulierten Streifens der Mucosa eine einheitlichere, im therapeutischen Sinne einheitliche Wirkungstiefe ergibt. Damit lässt sich eine gleichmäßig und großflächige Gewebeablation erreichen.

Das erfindungsgemäße Konzept ebnet den Weg zu der Gestaltung eines besonders schmalen, schlanken Kopfs der kaum breiter oder im Einzelfall auch schmaler sein kann als die von ihm erzeugte Spur koagulierten Mucosagewebes. Dies ermöglicht wiederum eine Vereinfachung der Handhabung und den Anbau der Ablationsvorrichtung an einem Endoskop, insbesondere das Befestigen der Ablationsvorrichtung an dem distalen Ende des Endoskops, wodurch dessen Kanäle für weitere Instrumente sowie zur Absaugung von Rauch frei bleiben. Das Endoskop sowie die aufgesetzte Ablationsvorrichtung können in einem Folienschlauch angeordnet sein. Dieser kann zwei getrennte Lumen aufweisen, so dass das Endoskop durch ein Lumen und die Ablationsvorrichtung durch das zweite Lumen geführt wird. In das Lumen für die Ablationsvorrichtung kann ein Führungselement, beispielsweise ein Spiralschlauch eingesetzt sein, in welchem die Ablationsvorrichtung verschiebbar ist.

Die Verschiebbarkeit des Kopfs der Ablationsvorrichtung in Axialrichtung, d.h. in Längsrichtung des Endoskops, erleichtert die Handhabung insbesondere bei der Ablation von Gewebeschichten in großen Hohlorganen, wie beispielsweise Ablation der Mucosa im Magen. Der Kopf der Vorrichtung kann zum Beispiel zwischen 0 mm und 100 mm ausgefahren werden, wobei eine Ausfahrbarkeit bis 50 mm als vorteilhaft, weiter werden 30mm Ausfahrlänge als optimal angesehen.

Es wird als besonders vorteilhaft angesehen, wenn der Kopf an seinem distalen Ende einen unrunden Querschnitt aufweist und die ihm zugeordnete Fassung eine entsprechende komplementäre Ausnehmung aufweist, in die der Kopf bei Rückzug in proximaler Richtung einfahren kann. Der Kopf wird dadurch in der Fassung in seiner Drehbeweglichkeit eingeschränkt. Dies erleichtert die Handhabung der Ablationsvorrichtung erheblich. Insbesondere kann sichergestellt werden, dass der Kopf durch Rückzug in einer bestimmten Drehlage angeordnet ist, die er auch nach dem Ausschieben beibehält. Der Kopf kann vorteilhaft auch in dem ausgefahrenen Zustand durch eine Erhebung am Schlauch am proximalen Ende der Fassung durch eine dafür vorgesehene Nut in einer gewünschten Lage gebracht werden.

An dem Kopf kann neben einem oder mehreren Gasleitungskanälen auch ein Fluidleitungskanal angeordnet sein, der zum Ausstoßen eines Fluidstrahls beispielsweise eines Wasserstrahls dienen kann. Die Mündung dieses Kanals ist vorzugsweise zwischen den Mündungen der Gaskanäle angeordnet. Es kann ein Flüssigkeitsstrahl mit einem zum Beispiel zum Unterspritzen der Mucosa erforderlichen Druck bzw. Flow ausgebildet werden.

Die Gaskanäle sind vorzugsweise mit einer keramischen Auskleidung versehen. Dies ermöglicht Dauerbetrieb der Ablationsvorrichtung, insbesondere zum Abtragen größerer Bereiche der Mucosa.

Die beiden Elektroden sind vorzugsweise elektrisch gegeneinander isoliert und an gesonderte Zuleitungen angeschlossen. Die beiden Zuleitungen können durch einen gemeinsamen Gaszuführungskanal oder durch getrennte Gaszuführungskanäle geführt sein. Vorzugsweise werden die beiden Elektroden über die gesonderten Leitungen abwechselnd mit elektrischer Leistung beaufschlagt, wodurch sich ein besonderer gleichmäßiger Ablationseffekt und eine, über die Breite des erzeugten Ablationsstreifens gemessen, einheitliche Koagulationstiefe erreichen lässt. Dies insbesondere, wenn an den beiden Elektroden alternierend hochfrequente Spannung im Bereich mehrerer Hundert kHz anliegt, wobei die Umschaltfrequenz (Alternierungsfrequenz) zwischen beiden Elektroden wenige Hz beträgt. Die an die Elektroden abgegebene Leistung kann beispielsweise zwischen 10 W und 200 W, beispielsweise 80 W oder 120 W betragen. Weitere Einzelheiten vorteilhafter Ausführungsformen der Erfindung sind Gegenstand der Beschreibung, der Zeichnung oder von Ansprüchen. Es zeigen:
Figur 1 eine erfindungsgemäße Ablationsvorrichtung an einem Endoskop im Einsatz, in schematischer Darstellung,
Figur 2 ein Ende eines Endoskops mit darauf aufgesetzter Ablationsvorrichtung, in ausschnittsweiser Seitenansicht,
Figur 3a/ 3b/ 3c das Endoskop mit der Ablationsvorrichtung nach Figur 2, in perspektivischer Ansicht,
Figur 4 das Endoskop und die Ablationsvorrichtung nach Figur 2 und 3, in Stirnansicht,
Figur 5 die Ablationsvorrichtung, in ausschnittsweiser Schnittdarstellung,
Figur 6 die elektrische Beschaltung der Ablationsvorrichtung, als Schaltschema, und
Figur 7 eine abgewandelte Ausführungsform einer Ablationsvorrichtung in schematischer, längsgeschnittener Darstellung in Betrieb bei Einwirkung auf die Mucosa.

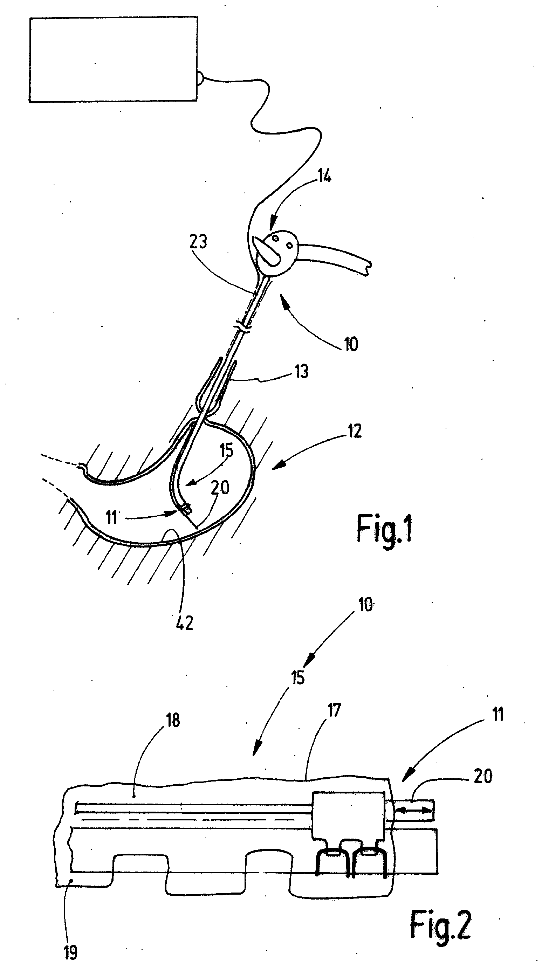

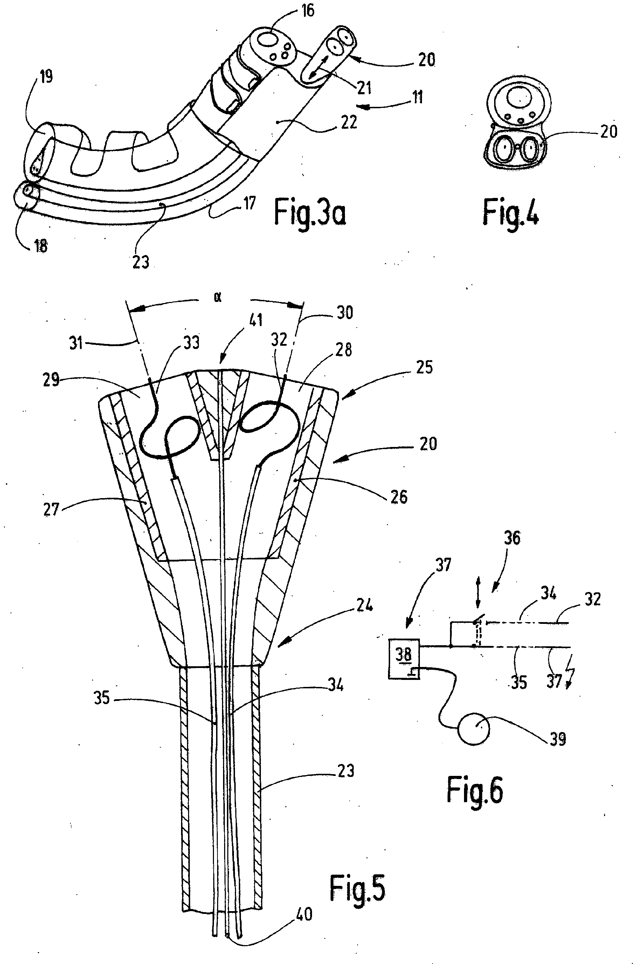

In Figur 1 ist die erfindungsgemäße, an einem Endoskop 10 angebrachte Ablationsvorrichtung 11 während des Vorgangs der Mucosaablation an einem Magen 12 veranschaulicht. Zur Behandlung werden das Endoskop 10 und die Ablationsvorrichtung 11 durch die Speiseröhre 13 in den Innenraum des Magens 12 eingefügt. Bedienelemente 14 gestatten dabei die Bewegung des Endoskops 10 so, dass sich beispielsweise ein distales Ende 15 des Endoskops 10 gezielt krümmen und/oder entlang der Mageninnenwand bewegen lässt, wodurch verschiedene Stellen der Mageninnenwand leicht erreichbar sind. Das Endoskop 10 kann ein oder mehrere Kanäle 16 (siehe Fig.3a) enthalten, in die Arbeitsmittel, wie beispielsweise chirurgische Instrumente, eingeführt werden können und über die gasförmige und flüssige Fluide zu oder abgeführt werden können. Außerdem kann das Endoskop 10 Mittel zur Bildübertragung enthalten, um die Behandlung optisch kontrollieren zu können.

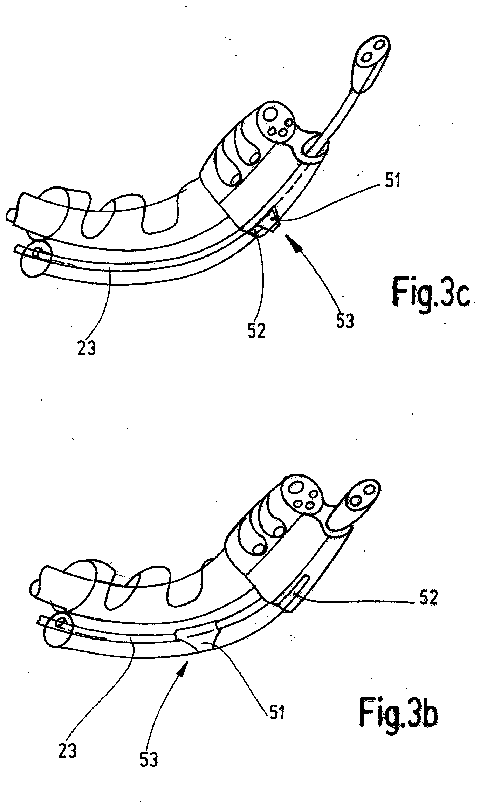

Die Figuren 2 bis 4 veranschaulichen das distale Ende 15 des Endoskops 10 mit der daran befestigten Ablationsvorrichtung 11. Diese ist gemeinsam mit dem Endoskop 10 in einer Schlauchhülle 17 angeordnet, die ein erstes Lumen 18 für die Ablationsvorrichtung 11 und ein zweites Lumen 19 für das Endoskop 10 enthält. Das erste und/oder das zweite Lumen 18, 19 können, wie in Figur 2 und 3 veranschaulicht, geschlitzt ausgebildet sein, um ein Krümmen des Endoskops zu erleichtern. Die Schlauchhülle 17 kann aus einer dünnen Kunststofffolie bestehen, die das Endoskop 10 und die Ablationsvorrichtung 11 mit entsprechendem Spiel umschließt.

Die Ablationsvorrichtung 11 ist im vorliegenden Ausführungsbeispiel als an dem Endoskop 10 außen angebrachte, sich längs desselben erstreckende Vorrichtung ausgebildet. Die Erfindung ist darauf jedoch nicht beschränkt. Die Ablationsvorrichtung kann auch als innerhalb eines Endoskops geführte Vorrichtung ausgebildet sein. Jedenfalls weist sie einen Kopf 20 auf, der vorzugsweise beweglich gelagert, vorzugsweise in Axialrichtung 21 beweglich ist, die mit der Längsrichtung des distalen Endes des Endoskops 10 übereinstimmt. Dem Kopf 20 ist eine Fassung 22 zugeordnet, die in oder an dem Endoskop 10 gehalten ist. Die Fassung 22 weist eine Durchgangsöffnung auf, in die der Kopf 20 einfahren kann und durch die sich ein Versorgungsschlauch 23 für den Kopf 20 erstreckt. Der Versorgungsschlauch 23 ist vorzugsweise zug- und drucksteif, jedoch leicht biegbar. Deswegen kann der Kopf 20 in Axialrichtung 21 hin und her bewegt werden, indem der Schlauch 23 entsprechend hin und her geschoben bzw. gezogen wird.

Der Kopf 20 ist in Figur 4 in Draufsicht veranschaulicht und in Figur 5 gesondert im Längsschnitt dargestellt. Es ist ersichtlich, dass der Kopf 20 einen von der Kreisform abweichenden Querschnitt aufweist, insbesondere einen Ovalquerschnitt. Jedenfalls sind die Abweichung des Querschnitts der Durchgangsöffnung der Fassung 22 und die Form des Kopfes 20 derart, dass der Kopf 20 beim Rückziehen in die Durchgangsöffnung der Fassung 22 findet und sich dabei in die gewünschte Winkelposition dreht. Um dies zu bewirken, weist der Kopf 20 vorzugsweise an seinem proximalen Ende 24 einen Kreisquerschnitt auf. Er ist dort zum Beispiel als Kreiskegel ausgebildet. Der Querschnitt des Kegels geht dann ausgehend vom proximalen Ende 24 in Richtung des distalen Endes 25 allmählich in die aus Figur 4 ersichtliche Ovalform über, wobei die Außenfläche des Kopf 20 frei von Stufen ist, die an der Mündung der Durchgangsöffnung der Fassung 22 aufsetzen und somit das Rückziehen des Kopfs 20 behindern könnten.

Diese Bauform trägt erheblich zur Handhabungserleichterung der Ablationsvorrichtung 11 bei. Wird der Kopf 20 weit aus der Fassung 20 herausgeschoben, so dass lediglich noch das proximale Ende 24 in der Fassung 22 steht oder dass der Kopf 20 ganz aus der Fassung 20 rausgeschoben ist, kann der Kopf 20 über ein Drehen des relativ steifen Versorgungsschlauchs 23 in verschiedene Winkelpositionen gedreht werden. Wird der Kopf 20 jedoch zurückgezogen, findet er in die Fassung 22 ein, die den Kopf 20 dann in die vorgegebene Winkelposition dreht. Die Überführung des Kopfs 20 in die in Figur 3 und 4 veranschaulichte Sollposition ist somit automatisch durch Formschluss zwischen dem Kopf 20 und der Fassung 22 gegeben. Die Form des Kopfs 20 und der Fassung 22 sind so aufeinander abgestimmt, dass der Kopf 20 nicht nur in seiner Endposition, wenn er ganz in die Fassung 22 eingezogen ist, in radialer Richtung fixiert ist, sondern diese Orientierung im Wesentlichen beibehält, bis der Kopf 20 komplett aus der Fassung 22 austritt. Dadurch ist es möglich, den Kopf 20 in axialer Richtung zu verschieben, und trotzdem behält er seine Rotationsorientierung im Wesentlichen bei. Die Länge der axialen Verschiebung, wobei der Kopf 20 seine Position in radialer Richtung im Wesentlichen beibehält, beträgt 15 mm, vorzugsweise 10 mm, besonders vorzugsweise 8 mm. Der Kopf 20 kann aus einem hitzebeständigen, elektrisch nicht und thermisch wenig leitenden Material bereitgestellt sein, wie beispielsweise hitzebeständigem Kunststoff, Duroplast, Keramik oder dergleichen. Es ist auch möglich, den Kopf 20, wie in Figur 5 angedeutet, aus Kunststoff auszubilden und mit keramischen Einlagen 26, 27 beispielsweise in Rohrform zu versehen. Der Kopf kann auch im Ganzen aus Keramik ausgebildet sein.

Um die Orientierung des Kopfs 20 in radialer Richtung sicherzustellen, wenn der Kopf 20 in axialer Richtung soweit bewegt worden, dass er aus der Fassung 22 austritt, kann der Versorgungschlauch 23 mit einer Verdrehsicherung 53 gemäß Figur 3b und 3c ausgebildet sein. Diese Verdrehsicherung 53 weist dazu eine vorstehende Formnase in Form einer Feder 51 auf. Diese Feder 51 taucht in eine an der Fassung 22 vorgesehene Nut 52 ein, und sichert somit im ausgefahrenen Zustand des Kopfs 20 die gewünschte Lage (Figur 3c). Die Verdrehsicherung 53 kann beispielsweise als Kunststoffumspritzung ausgeführt sein. Die Verdrehsicherung 53 kann so ausgebildet sein, dass sie zusammen mit der Fassung 22 einen Endanschlag bildet, der die maximale Länge begrenzt, die der Kopf 20 in axialer Richtung bewegt werden kann. Es ist möglich, die Verdrehsicherung 53 in einem Abstand zur Fassung 22 am Versorgungsschlauch 23 anzuordnen, so dass dadurch die Rotationsorientierung des Kopfs 20 in jeder Axialposition sichergestellt ist. Dazu ist es dann erforderlich, dass kurz vor dem Verlassen des Kopfs 20 aus der Fassung 22 die Feder 51 bereits in die Nut 52 eingreift oder wenigstens teilweise eingreift.

Es ist auch möglich, die Verdrehsicherung 53 so anzuordnen, dass der Kopf 20 zwischen seinen Endbereichen in Rotationsrichtung frei beweglich gehalten ist. Bei solch einer Anordnung ist die Rotationsfixierung des Kopfs 20 solange gewährt, bis er die Fassung 22 verlässt, und dann wieder, wenn die Feder 51 in die Nut 52 eintritt. Bei einer gesamten Axialbewegung des Kopfs 20 von beispielsweise 50 mm kann die fixierte Rotationsrichtung des Kopfs 20 in den Bereichen seiner jeweiligen Endlagen jeweils ca. 15 mm einnehmen. Dazwischen kann der Kopf 20 ca. 20 mm auch in radialer Richtung frei beweglich gehalten sein.

Der Kopf 20 umschließt wenigstens zwei Kanäle 28, 29, die dazu dienen, dem Behandlungsort Gas, insbesondere Inertgas wie beispielsweise Argon, zuzuführen. Es können jedoch auch Aktivgase, Aerosole oder dergleichen zuzuführen sein, wozu die Kanäle 28, 29 gleichermaßen dienen können. Im vorliegenden Ausführungsbeispiel werden die Kanäle 28, 29 über den einzigen Versorgungsschlauch 23 gemeinsam mit Gas gespeist. Die Kanäle 28, 29 können einen Kreisquerschnitt oder auch abweichende Querschnitte wie Ovalquerschnitt, Mehreckquerschnitte oder dergleichen aufweisen. An ihren Öffnungen weisen sie unterschiedlich orientierte Öffnungsachsen 30, 31 auf, die in Figur 5 durch strichpunktierte Linien markiert sind, die Öffnungsachsen sind die Mittelachsen der Öffnungen der Kanäle 28, 29 und schließen miteinander einen Winkel α ein, der vorzugsweise im Bereich zwischen 10 und 60°, vorzugsweise zwischen 10° und 30° liegt und im bevorzugten Fall 25° beträgt. Die Kanäle 28, 29 können auch zu einem gemeinsamen Kanal mit Ovalquerschnitt oder Schlitzquerschnitt vereinigt sein, in dem beide Elektroden 32, 33 angeordnet sind.

Der Abstand zwischen den Elektroden 32, 33 beträgt vorzugsweise mehrere Millimeter (3 mm bis 12 mm), wobei ein Abstand von 5 mm bis 10 mm, insbesondere 7,5 mm vorteilhaft ist, um eine gleichmäßige breitstreifige Gewebeablation mit homogener Ablationstiefe zu erreichen. Der Durchmesser der Elektroden liegt vorzugsweise im Bereich von 0,2 mm bis 1 mm, wobei im vorliegenden Ausführungsbeispiel ein Durchmesser von 0,4 mm gewählt worden ist. Dies hat sich wegen der aufgrund des geringen Drahtdurchmessers auftretenden hohen Feldstärken und deswegen der guten Zündwilligkeit der Elektroden einerseits und der damit zu erzielenden Gewebeeffekte anderseits als vorteilhaft herausgestellt.

Vorzugsweise mittig in den Öffnungen der Kanäle 28, 29 sind stab- oder nadelförmige Elektroden 32, 33 angeordnet, die längs zu den Öffnungsachsen 30, 31 orientiert sind. Die Spitzen der Elektroden 32, 33 können innerhalb der Kanäle 28, 29 liegen oder, wie es Figur 5 andeutet, aus diesen herausschauen. Die Elektroden 32, 33, die beispielsweise aus einem hitzebeständigen Metall wie Wolfram ausgebildet sein können, sind durch geeignete Mittel, in Figur 5 symbolisch angedeutet durch eine federnde Leiterschleife, etwa zentrisch in den Kanälen 28, 29 gehalten. An die Elektroden 32, 33 sind Zuleitungen 34, 35 angeschlossen, die gegeneinander elektrisch isoliert sind und durch den Versorgungsschlauch 23 führen. Wie Figur 6 veranschaulicht, sind die Leitungen 34, 35 über eine Schalteranordnung 36 mit einer elektrischen Quelle 37 zum Beispiel in Gestalt eines Hochfrequenzgenerators 38 angeschlossen. Dieser stellt eine HF-Spannung mit mehreren hundert kHz (zum Beispiel 350 kHz) und einer geeigneten Spannung oberhalb 1000 V (z.B. 4500 V) bereit. Der HF-Generator 38 kann eine Leistung vom mehr als 100 W (zum Beispiel 120 W) aufbringen.

Die Spannung wird bezogen auf ein Null-Potential bereitgestellt, an das der Patient über mindestens eine Neutralelektrode 39 angeschlossen ist. Diese Neutralelektrode 39 wird großflächig an einer geeigneten Stelle des Körpers des Patienten angebracht. Die Schalteranordnung 36 verbindet die Leitungen 34, 35 und somit die Elektroden 32, 33 alternierend, d.h. abwechselnd mit dem Ausgang der elektrischen Quelle 37. Die Schaltfrequenz, mit der die Elektroden 32, 33 abwechselnd aktiviert werden, liegt im Bereich einiger Hz, vorzugsweise zwischen 1 Hz und 20 Hz, vorzugsweise 5 Hz.

Zusätzlich kann sich eine Fluidleitung 40 durch den Versorgungsschlauch 23 erstrecken und mit einer an der Stirnseite des Kopfs 20 vorgesehenen Düse verbunden sein. Die Düse weist an ihrem distalen Ende eine Austrittsöffnung 41 auf durch die eine Flüssigkeit, zum Beispiel Natriumchlorid-Lösung, beispielweise als Strahl ausgestoßen werden kann. Damit lassen sich Gewebepartien bearbeiten, beispielsweise unterspritzen, wenn der Strahl mit entsprechendem Druck, Flow und Form austritt, dass er wie eine Nadel in das Gewebe eindringen kann.

Die insoweit beschriebene Ablationsvorrichtung 11 arbeitet wie folgt: Zur flächenhaften Ablation der Mucosa, beispielsweise zur therapeutischen Behandlung krankhafter Gewebeveränderungen, zur Beeinflussung des Gewichts- und Essverhaltens von Patienten oder aus anderen therapeutischen Gründen, wird das mit der Ablationsvorrichtung 11 versehene Endoskop 10 gemäß Figur 1 durch die Speiseröhre 13 des Patienten in dessen Magen 12 eingeführt. Mittels der Bedienelemente 14 des Endoskops 10 wird dessen distales Ende 15 an die gewünschte Ablationsstelle so positioniert, dass die zu behandelnde Gewebestelle im Sichtfeld des Endoskops 10 liegt. Es wird nun durch entsprechendes Schieben des Versorgungsschlauchs 13 der Kopf 20 der Ablationsvorrichtung 1 etwas vorgeschoben, so dass er im gewünschten Abstand, beispielsweise 3 mm zu der Mucosa 42 steht.

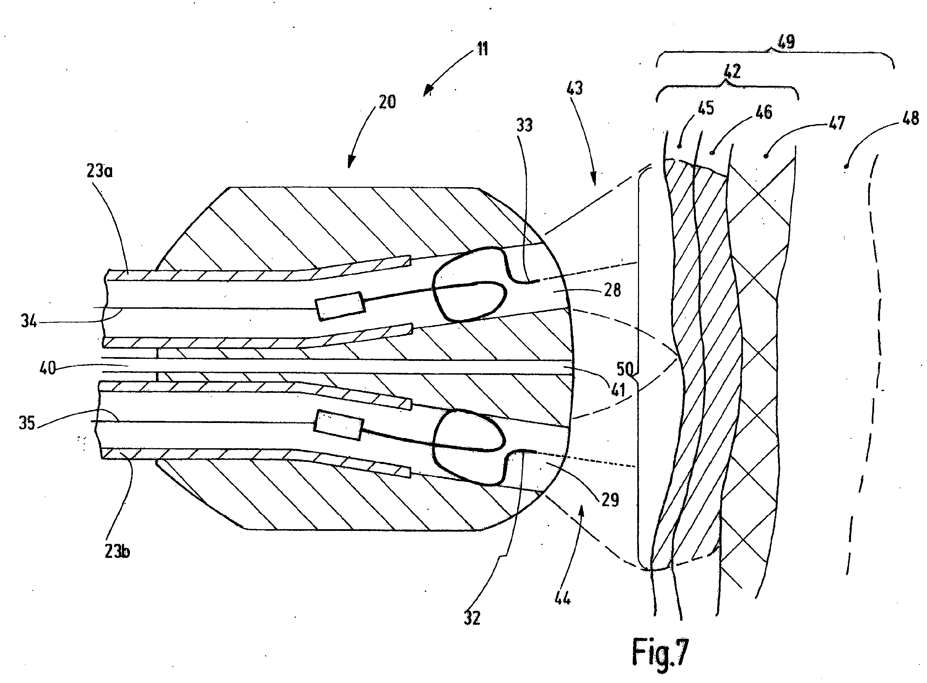

Vor thermischer Ablation beispielhaft der Mukosa 42 wird durch die Austrittsöffnung 41 Flüssigkeit in die Magenwand 49 so eingebracht, dass sich ein Flüssigkeitskissen vorteilhaft unter der gewünschten Ablationsstelle bildet. Durch die Kanäle 28, 29 strömt Gas, beispielsweise Argon. Es werden nun der Generator 38 und die Schalteranordnung 36 aktiviert, so dass die Elektroden 32, 33 abwechselnd zünden und einen Funken zur Mucosa 42 überspringen lassen. Der einhüllende Argonstrahl bildet vor jeder Elektrode 30, 31 einen Plasmastrahl 43, 44, der am Beispiel der Ausführungsform nach Figur 7 ersichtlich ist. Sie können sich dabei zu einem fächerförmigen Strahl vereinigen. Die Plasmastrahlen 43, 44 treffen zeitlich versetzt nebeneinander auf der Mucosa 42 auf und koagulieren deren oberste Schicht, insbesondere deren Epithel 45 sowie der Lamina Propria 46 und Teile der Submucosa 47. Die Muscularis Propria 48 wird jedoch durch zuvor gebildete das Flüssigkeitskissen vorzugsweise verschont.

Durch das abwechselnde Ansteuern der beiden Plasmastrahlen mit 5 Hz kommt es makroskopisch zur Vereinigung der Plasmastrahlen 43, 44. So wird ein breiter Gewebestreifen 50 mit einheitlicher Wirktiefe koaguliert. Die Breite des Gewebestreifens 50 kann durch die Winkelanordnung der Elektroden 32, 33 und der Kanäle 28, 29 über 10 mm und im Einzelfall etwa 14 mm betragen. Die Behandlung nimmt ihren Fortgang, indem der Anwender mittels der Bedienelemente 14 des Endoskops 10 und durch entsprechende Führung des Versorgungsschlauchs 23 den Kopf 20 entlang eines Wegs über die Mucosa führt (in Figur 7 senkrecht zur Zeichenebene), wobei er einen koagulierten Streifen Gewebes von etwa 12 mm bis 14 mm Breite hinterlässt. Auf diese Weise kann die Mucosa 42 in großer Zuverlässigkeit mit reduzierter Gefahr der Muscularisschädigung koaguliert werden.

Figur 7 veranschaulicht eine abgewandelte Ausführungsform der Ablationsvorrichtung 11. Der Unterschied besteht in der Ausbildung des Kopfs 20, dessen Kanäle 28, 29 von gesonderten Versorgungsschläuchen 23a, 23b gespeist werden. Entsprechend können die Leitungen 34, 35 ohne gesonderte Isolierung in diesen Versorgungschläuchen 23a, 23b geführt werden. Im Übrigen gilt für diese Ausführungsform die obige Beschreibung entsprechend unter Zugrundelegung gleicher Bezugszeichen.

Die erfindungsgemäße Ablationsvorrichtung 11 zeichnet sich durch einen Kopf 20 mit unrundem Querschnitt und in einem spitzen Winkel zueinander stehenden Elektroden 32, 33 aus. Durch die Winkelanordnung der Elektroden und entsprechende Ausbildung der Kanäle 28, 29 ergibt sich ein etwa fächerförmiger Plasmastrahl mit dem insbesondere mit alternierender Aktivierung der beiden Elektroden ein breiter streifenförmiger Ablationsbereich z.B. Hohlorganen von Lebewesen erzielbar ist. Die Handhabung ist zuverlässig und vereinfacht und die Behandlungszeit im Vergleich zu vorhanden Maßnahmen verkürzt.

### Bezugszeichen:

| | |
|---|---|
| 10 | Endoskop |
| 11 | Ablationsvorrichtung |
| 12 | Magen |
| 13 | Speiseröhre |
| 14 | Bedienelemente |
| 15 | distales Ende des Endoskops 10 |
| 16 | Kanal des Endoskops 10 |
| 17 | Schlauchhülle |
| 18 | erstes Lumen der Schlauchhülle 17 |
| 19 | zweites Lumen der Schlauchhülle 17 |
| 20 | Kopf |
| 21 | Axialrichtung |
| 22 | Fassung |
| 23, 23a, 23b | Versorgungsschlauch |
| 24 | proximales Ende des Kopfs 20 |
| 25 | distales Ende des Kopfs 20 |
| 26, 27 | keramische Einlagen |
| 28, 29 | Kanäle |
| 30, 31 | Öffnungsachsen |
| α | Winkel zwischen den Öffnungsachsen 30, 31 |
| 32, 33 | Elektroden |
| 34, 35 | Leitungen |
| 36 | Schalteranordnung |
| 37 | elektrische Quelle |
| 38 | HF-Generator |
| 39 | Neutralelektrode |
| 40 | Fluidleitung |
| 41 | Austrittsöffnung |
| 42 | Mucosa |
| 43, 44 | Plasmastrahl |
| 45 | Epithel |
| 46 | Lamina Propria |
| 47 | Submucosa |
| 48 | Muscularis Propria |
| 49 | Magenwand |
| 50 | Gewebestreifen |
| 51 | Formnase, Feder |
| 52 | Nut, Schlitz |
| 53 | Verdrehsicherung |
| | |

## Patentansprüche

1. Ablationsvorrichtung (11), insbesondere für die Mucosaablation,
mit einem Kopf (20), der einen ersten Kanal (28) mit einer wenigstens teilweise darin angeordneten ersten Elektrode (32) und einen zweiten Kanal (29) mit einer wenigstens teilweise darin angeordneten zweite Elektrode (33) aufweist,
wobei der erste Kanal (28) und der zweite Kanal (29) miteinander und die erste Elektrode (32) und die zweite Elektrode (33) miteinander einen spitzen Winkel (α) einschließen,
**dadurch gekennzeichnet, dass** dadurch die von den beiden Kanälen (28, 29) ausgehenden Plasmaströme divergieren.

2. Ablationsvorrichtung nach Anspruch 1, wobei der erste Kanal (28) und der zweite Kanal (29) Gasleitungskanäle sind.

3. Ablationsvorrichtung nach einem der vorstehenden Ansprüche, wobei die Kanäle (28, 29) jeweils eine gerundete Mündung mit zentrisch in dieser platzierten Elektrode (32, 33) aufweisen.

4. Ablationsvorrichtung nach einem der vorstehenden Ansprüche, wobei die Kanäle (28, 29)' zumindest an ihren distalen Enden aus einem hitzebeständigen Kunststoff oder Keramik ausgebildet sind.

5. Ablationsvorrichtung nach einem der vorstehenden Ansprüche, wobei der erste Kanal (28) und der zweite Kanal (29) an eine gemeinsame Versorgungsleitung (23) angeschlossen sind.

6. Ablationsvorrichtung nach einem der Ansprüche 1 bis 4, wobei der erste Kanal (28) und der zweite Kanal (29) an gesonderte Versorgungsleitungen (23a, 23b) angeschlossen sind.

7. Ablationsvorrichtung nach einem der vorstehenden Ansprüche, wobei sich durch die Versorgungsleitung (23, 23a, 23b) eine elektrische Zuleitung (34, 35) für wenigstens eine der Elektroden (32, 33) erstreckt.

8. Ablationsvorrichtung nach Anspruch 7, wobei die elektrischen Zuleitungen (34, 35) der beiden Elektroden (32, 33) gegeneinander elektrisch isoliert sind.

9. Ablationsvorrichtung nach einem der vorstehenden Ansprüche, wobei der Kopf (20) sich ausgehend von seinem distalen Ende (25) in proximaler Richtung verjüngend ausgebildet ist.

10. Ablationsvorrichtung nach einem der vorstehenden Ansprüche, wobei der Kopf (20) an seinem distalen Ende (25) einen unrunden Querschnitt aufweist.

11. Ablationsvorrichtung nach einem der vorstehenden Ansprüche, wobei dem Kopf (20) eine Fassung (22) mit einer Halteeinrichtung zu Befestigung an einem Endoskop (10) zugeordnet ist, wobei der Kopf (20) in Bezug auf die Fassung (22) axial beweglich ist.

12. Ablationsvorrichtung nach Anspruch 9, 10 und 11, wobei die Drehbeweglichkeit des Kopfs (20) in der Fassung (22) eingeschränkt ist.

13. Ablationsvorrichtung nach einem der vorstehenden Ansprüche, wobei an dem Kopf (20) eine Austrittsöffnung (41) für eine Flüssigkeit angeordnet ist.

14. Ablationsvorrichtung nach Anspruch 13, wobei die Austrittsöffnung (41) eine Düse ist.

15. Ablationsvorrichtung nach Anspruch 13 oder 14, wobei die Austrittsöffnung (41) mittig zwischen den Elektroden (32, 33) angeordnet ist.

## Claims

1. Ablation device (11), in particular for mucosal ablation,
with a head (20) comprising a first channel (28) with a first electrode arranged at least partially therein and comprising a second channel (29) with a second electrode (33) arranged at least partially therein,
wherein the first channel (28) and the second channel (29) together and the first electrode (32) and the second electrode (33) together, include an acute angle (α)
**characterized in that** the plasma streams originating from the two channels (28, 29) diverge.

2. Ablation device as in Claim 1, wherein the first channel (28) and the second channel (29) are gas-conducting channels.

3. Ablation device as in one of the previous claims, wherein each of the channels (28, 29) has a rounded mouth with an electrode (32, 33) centrally placed therein.

4. Ablation device as in one of the previous claims, wherein the channels (28, 29), at least on their distal ends, are made of a heat-resistant plastic or ceramic material.

5. Ablation device as in one of the previous claims, wherein the first channel (28) and the second channel (29) are connected to a common supply line (23).

6. Ablation device as in one of the claims 1 to 4, wherein the first channel (28) and the second channel (29) are connected to separate supply lines (23a, 23b).

7. Ablation device as in one of the previous claims, wherein an electrical supply line (34, 35) for at least one of the electrodes (32, 33) extends through the supply line (23, 23a, 23b).

8. Ablation device as in Claim 7, wherein the electrical supply lines (34, 35) of the two electrodes (32, 33) are electrically insulated with respect to each other.

9. Ablation device as in one of the previous claims, wherein the head (20) is configured so as to taper in proximal direction starting from its distal end (25).

10. Ablation device as in one of the previous claims, wherein the head (20) has a non-circular cross-section on its distal end (25).

11. Ablation device as in one of the previous claims, wherein a socket (22) having a holding device for attachment to an endoscope (10) is assigned to the head (20), wherein the head (20) is axially moveable relative to the socket (22).

12. Ablation device as in Claims 9, 10 and 11, wherein the rotatability of the head (20) in the socket (22) is restricted.

13. Ablation device as in one of the previous claims, wherein a discharge opening (41) for a fluid is arranged on the head (20).

14. Ablation device as in Claim 13, wherein the discharge opening (41) is a nozzle.

15. Ablation device as in Claim 13 or 14, wherein the discharge opening (41) is arranged so as to be centered between the electrodes (32, 33).

## Revendications

1. Dispositif d'ablation (11), destiné en particulier à l'ablation de muqueuse,
comprenant une tête (20) qui présente un premier canal (28) avec une première électrode (32), disposée au moins en partie dans celui-ci, et un deuxième canal (29) avec une deuxième électrode (33), disposée au moins en partie dans celui-ci,
le premier canal (28) et le deuxième canal (29) formant entre eux, et la première électrode (32) et la deuxième électrode (33) formant entre elles un angle aigu (a), **caractérisé en ce que** de ce fait, les flux de plasma partant des deux canaux (28, 29) sont divergents.

2. Dispositif d'ablation selon la revendication 1, dans lequel le premier canal (28) et le deuxième canal (29) sont des canaux de conduite de gaz.

3. Dispositif d'ablation selon l'une des revendications précédentes, dans lequel les canaux (28, 29) présentent respectivement une bouche arrondie, dans laquelle l'électrode (32, 33) est disposée de façon centrée.

4. Dispositif d'ablation selon l'une des revendications précédentes, dans lequel les canaux (28, 29) sont réalisés au moins à leurs extrémités distales à partir d'une matière plastique résistant à la chaleur ou à partir de céramique.

5. Dispositif d'ablation selon l'une des revendications précédentes, dans lequel le premier canal (28) et le deuxième canal (29) sont raccordés à une conduite d'alimentation (23) commune.

6. Dispositif d'ablation selon l'une des revendications 1 à 4, dans lequel le premier canal (28) et le deuxième canal (29) sont raccordés à des conduites d'alimentation (23a, 23b) distinctes.

7. Dispositif d'ablation selon l'une des revendications précédentes, dans lequel une ligne d'alimentation électrique (34, 35) pour au moins une des électrodes (32, 33) s'étend à travers la conduite d'alimentation (23 23a, 23b).

8. Dispositif d'ablation selon la revendication 7, dans lequel les lignes d'alimentation électriques (34, 35) des deux électrodes (32, 33) sont isolées électriquement l'une vis-à-vis de l'autre.

9. Dispositif d'ablation selon l'une des revendications précédentes, dans lequel la tête (20) est réalisée de manière à se rétrécir à partir de son extrémité distale (25), dans la direction proximale.

10. Dispositif d'ablation selon l'une des revendications précédentes, dans lequel la tête (20) présente une section transversale non ronde à son extrémité distale (25).

11. Dispositif d'ablation selon l'une des revendications précédentes, dans lequel un support (22), doté d'un dispositif de maintien pour la fixation à un endoscope (10), est associé à la tête (20), la tête (20) présentant une mobilité axiale par rapport au support (22).

12. Dispositif d'ablation selon les revendications 9, 10 et 11, dans lequel la mobilité rotatoire de la tête (20) dans le support (22) est limitée.

13. Dispositif d'ablation selon l'une des revendications précédentes, dans lequel une ouverture de sortie (41) pour un liquide est prévue sur la tête (20).

14. Dispositif d'ablation selon la revendication 13, dans lequel l'ouverture de sortie (41) est une buse.

15. Dispositif d'ablation selon la revendication 13 ou 14, dans lequel l'ouverture de sortie (41) est disposée au milieu entre les électrodes (32, 33).
